# EUROPEAN PATENT APPLICATION

(11) **EP 2 401 957 A1**
(43) Date of publication of application: **04.01.2012**
(21) Application number: 11169680.3
(22) Date of filing: 13.06.2011
(51) Int. Cl.: A61B 3/10, A61B 5/00, A61B 3/117, G01N 33/68, G01N 21/65, G01N 21/31

(54) **Raman spectral data analyzer, biological substance detection system, and biological substance detection method**

(30) Priority: 02.07.2010 JP 2010151721; 24.02.2011 JP 2011037790
(71) Applicant: Sony Corporation, Tokyo 108-0075 (JP)
(72) Inventor: Matsui, Eriko, Minato-ku, Tokyo 108-0075 (JP); Tamada, Sakuya, Minato-ku, Tokyo 108-0075 (JP); Kishimoto, Takuya, Minato-ku, Tokyo 108-0075 (JP); Yasuda, Akio, Minato-ku, Tokyo 108-0075 (JP)
(74) Representative: Horner, David Richard

(57) **Abstract**

Methods for early diagnosis of amyloid-related disorders based on the analysis of Raman spectra obtained from measurements carried out on body tissue (preferably on the eye lens) and spectral analyzers configured to carry out such methods.
The diagnosis is made by detection of a signature of amyloid beta in the spectral range of 1500-1800 cm-1. An intensity ratio is calculated and the presence or absence of amyloid beta is determined based on said ratio. The intensity ratio may either be the intensity ratio of a C-H band and an amide I band, or the intensity ratio of different waveform components of the amide I band, in which case the component decomposition is obtained curve fitting.

## Description

### FIELD

The present disclosure relates to spectral data analyzers, biological substance detection systems, and biological substance detection methods.

### BACKGROUND

Amyloid beta (amyloid β) is one of the main constituents of the neuritic plaques observed as the characteristic pathology in the brain of Alzheimer's disease patients. The amyloid beta is a polypeptide of about 40 amino acids, and cut out from the amyloid precursor protein (APP) by two types of secretases in the vicinity of the transmembrane region. In familial Alzheimer's disease patients, there has been report of a family line with APP point mutations, pointing to amyloid beta as a possible causative substance of Alzheimer's disease.

Amyloid beta accumulates in drusen, a characteristic pathology in the retina of patients with age-related macular degeneration, and is associated with the conditions of age-related macular degeneration, as reported in Drusen associated with aging and age-related macular degeneration contain proteins common to extracellular deposits associated with atherosclerosis, elastosis, amyloidosis, and dense deposit disease, FASEB J. 2000 May; 14 (7) :835-46. Age-related macular degeneration is a disease that causes blindness by atrophy or neovascularization in the macular region of the retina. Age-related macular degeneration is the No. 1 cause of adult blindness in Western and other developed countries. In Japan, the disease represents the leading cause of blindness after glaucoma along with diabetic retinopathy. Accordingly, there is a strong need for the establishment of a method for early diagnosis of age-related macular degeneration.

An assay system using a sandwich type enzyme-linked immunosorbent assay is available as an assay system for the detection of amyloid beta (see Isolation and quantification of soluble Alzheimer's beta-peptide from biological fluids, Nature. 1992 Sep 24; 359(6393):325-7).

Further, a detection method using Raman spectroscopy is available as a technique for detecting amyloid beta, as described in Roman signature from brain hippocampus could aid Alzheimer's disease diagnosis, Appl Opt. 2009 Aug 20;48(24) :4743-8. This publication attempts to provide ways to diagnose Alzheimer's disease through the Raman spectroscopy analysis of the frozen sections of hippocampus tissue removed from rats with Alzheimer's disease created by injecting amyloid beta to the CA1 region of the brain hippocampus. The publication describes the appearance of a characteristic peak at wavenumber 1670 cm⁻¹ in the amide I vibrational band in a Raman spectrum obtained from a diseases tissue. It is also reported that resolving the spectrum of the amide I vibrational band into multiple waveform components by curve fitting creates multiple waveform components of different peak wavenumbers in normal tissue, and a single main waveform component at peak wavenumber 1670 cm⁻¹ in a diseased tissue. From these findings, this publication concludes that detection of amyloid beta aggregation in a diseased tissue is possible by detecting a Raman peak at wavenumber 1670 cm⁻¹.

In connection with the present disclosure, apparatuses designed to measure or detect intraocular substances by Raman spectroscopy are available. For example, JP-A-10-272100 discloses an apparatus for measuring an intraocular substance by shining a visible to near infrared monochromatic or single-wavelength excitation light beam to an eye ball from an excitation optical system, and by detecting light that contains at least one of scattered light and fluorescence from the eye ball using an optical receiving system.

JP-T-2005-514137 discloses an apparatus that forms a Raman image of macular carotenoids (the term "JP-T" as used herein means a published Japanese translation of a PCT patent application). The apparatus produces an image that represents the spatial distribution and concentration of carotenoids, using components including a light source that generates light at a wavelength that produces a Raman response with a wavelength shift for carotenoids; a light delivery and collection means in optical communication with the light source for directing light onto tissue and collecting scattered light from the tissue; wavelength selective means for selecting Raman shifted light form collected scattered light; and detection means for measuring the intensity of the Raman shifted light at frequencies characteristic of the carotenoids.

### SUMMARY

Amyloid beta is associated with conditions of Alzheimer's disease or age-related macular degeneration, and thus a technique that can noninvasively detect amyloid beta accumulating in body tissue is considered useful for the early detection and the treatment of these diseases. Accordingly, it is desirable to provide a technique that can noninvasively detect substances in body tissue.

The present inventors found that accurate detection of amyloid beta in body tissue is possible by processing spectral data using a predetermined method after the spectrum representing the spectral data and that corresponds to a substance present in a body tissue is acquired by using Raman spectroscopy.

Based on this finding, an embodiment of the present disclosure provides a spectral data analyzer that calculates the spectral intensity ratio of a C-H band and an amide I band in a Raman spectrum that corresponds to a substance present in a body tissue, and that automatically determines the presence or absence of amyloid beta in the substance based on the calculated ratio. In the spectral data analyzer according to the embodiment of the present disclosure, the spectral intensity ratio may be the ratio of spectral intensities at wavenumbers 1463 cm⁻¹ and 1658 cm⁻¹ in the Raman spectrum. In the spectral data analyzer according to the embodiment of the present disclosure, the ratio may be calculated from multiple waveform components resolved from the Raman spectrum by curve fitting.

Another embodiment of the present disclosure provides a spectral data analyzer that resolves an amide I band of a Raman spectrum corresponding to a substance present in a body tissue into multiple waveform components by curve fitting, and calculates the ratio of (i) spectral intensities of waveform components that have peak wavenumbers in a 1635 to 1700 cm⁻¹ wavenumber range and (ii) spectral intensities of waveform components that have peak wavenumbers in a 1635 to 1655 cm⁻¹ wavenumber range, and that automatically determines the presence or absence of amyloid beta in the substance based on the calculated ratio.

Still another embodiment of the present disclosure provides a biological substance detection system that includes: a measurement device that acquires a Raman spectrum that corresponds to a substance present in a body tissue; and the spectral data analyzer of the embodiment of the present disclosure.

With the apparatuses, the amyloid beta present in a body tissue can be noninvasively detected with improved accuracy based on the calculated ratio.

Yet another embodiment of the present disclosure provides a biological substance detection method that includes: calculating the spectral intensity ratio of a C-H band and an amide I band in a Raman spectrum that corresponds to a substance present in a body tissue; and determining the presence or absence of amyloid beta in the substance based on the calculated ratio.

Still yet another embodiment of the present disclosure provides a biological substance detection method that includes: resolving an amide I band of a Raman spectrum corresponding to a substance present in a body tissue into multiple waveform components by curve fitting, and calculating the ratio of (i) spectral intensities of waveform components that have peak wavenumbers in a 1635 to 1700 cm⁻¹ wavenumber range and (ii) spectral intensities of waveform components that have peak wavenumbers in a 1635 to 1655 cm⁻¹ wavenumber range; and determining the presence or absence of amyloid beta in the substance based on the calculated ratio.

As used herein, "body tissue" includes retina and brain tissues, and various other tissues from the nerve, blood vessel, skin, stomach, small intestine, kidneys, and bodily fluid.

According to the embodiments of the present disclosure, a technique is provided that noninvasively detects a substance in body tissue.

Embodiments of the present disclosure provide a spectral data analyzer, among others, that uses Raman spectroscopy to detect the amyloid beta that accumulates in body tissue.

Further particular and preferred aspects of the present invention are set out in the accompanying independent and dependent claims. Features of the dependent claims may be combined with features of the independent claims as appropriate, and in combinations other than those explicitly set out in the claims.

### BRIEF DESCRIPTION OF THE DRAWINGS

The present invention will be described further, by way of example only, with reference to preferred embodiments thereof as illustrated in the accompanying drawings, in which:
FIGS. 1A and 1B are graphical representations explaining an example of Raman spectra obtained from a body tissue that contains amyloid beta (FIG. 1A) and from a body tissue that does not contain amyloid beta (FIG. 1B).
FIG. 2 is a graphical representation explaining an example of waveform components of a Raman spectrum obtained from a body tissue that contains amyloid beta.
FIG. 3 is a graphical representation explaining an example of waveform components of a Raman spectrum obtained from a body tissue that does not contain amyloid beta.
FIG. 4 is a spectrogram representing Raman spectra acquired in Test Example 1.
FIG. 5 is a spectrogram representing waveform components obtained by resolving the amide I vibrational band of a Raman spectrum of a tested eye.
FIG. 6 is a spectrogram representing a waveform component obtained by resolving the amide I vibrational band of a Raman spectrum of a control eye.
FIG. 7 is a Raman spectroscopic image of a tested eye.
FIG. 8 is a matrix representing a 1463 cm⁻¹/1658 cm⁻¹ ratio in each region in a Raman spectroscopic image of a tested eye.
FIG. 9 is a Raman spectroscopic image of a control eye.
FIG. 10 a matrix representing a 1463 cm⁻¹/1658 cm⁻¹ ratio in each region in a Raman spectroscopic image of a control eye.

### DETAILED DESCRIPTION

The following will describe embodiments of the present disclosure. It should be noted that the embodiments described below are examples of typical embodiments of the present disclosure, and should not be construed to limit the scope of the present disclosure. Descriptions will be given in the following order.
1. Spectral Data Analyzer and Biological Substance Detection System
   (1) First Embodiment
   (2) Second Embodiment
2. Biological Substance Detection Method

### 1. Spectral Data Analyzer and Biological Substance Detection System (1) First Embodiment

A biological substance detection system according to First Embodiment of the present disclosure is configured to include: a measurement device that acquires a Raman spectrum that corresponds to a substance present in a body tissue; and a spectral data analyzer that resolves the amide I band (vibrational band) of the acquired Raman spectrum into multiple waveform components by curve fitting, and calculates the ratio of (i) spectral intensities of waveform components that have peak wavenumbers in a 1635 to 1700 cm⁻¹ wavenumber range and (ii) spectral intensities of waveform components that have peak wavenumbers in a 1635 to 1655 cm⁻¹ wavenumber range, and that automatically determines the presence or absence of amyloid beta in the substance based on the calculated ratio.

More specifically, the spectral data analyzer according to the present embodiment calculates the ratio of the spectral intensities of the waveform components that have peak wavenumbers in the 1635 to 1655 cm⁻¹ wavenumber range with respect to the spectral intensities of the waveform components that have peak wavenumbers in the 1635 to 1700 cm⁻¹ wavenumber range, and determines and outputs that the substance contains amyloid beta, if the calculated ratio is equal to or greater than a predetermined value.

The biological substance detection system may be configured from a spectral data analyzer equipped with a program that causes a common computer including, for example, user interface (such as a display, a mouse, and a keyboard), a central processing unit (CPU), memory, and a storage unit (hard disc) to perform the procedure below, and a known Raman spectroscopy imaging device (measurement device).

The Raman spectroscopy imaging device is configured to include, for example, a light source, an irradiation system with which the light from the light source is guided and shone on a body tissue, and a detection system that selects and detects Raman shifted light from the scattered light generated from a substance in a body tissue in response to the irradiation light. The irradiation system and the detection system are configured from, for example, a condensing lens, an optical fiber, a dichroic mirror, a bandpass filter, and a PMT (photo multiplier tube).

The CPU, memory, and hard disc of the spectral data analyzer operate with the program stored in the hard disc to perform the following steps.

First, the Raman spectral data output from the Raman spectroscopy imaging device is processed by curve fitting algorithm to resolve the amide I vibrational band (1600-1700 cm⁻¹) of the Raman spectrum into multiple waveform components. The resolved waveform components potentially includes multiple waveform components that have peak wavenumbers in the 1635 to 1700 cm⁻¹ wavenumber range, and two waveform components that have peak wavenumbers in the 1635 to 1655 cm⁻¹ wavenumber range.

Then, calculations are performed to find the spectral intensities of the waveform components that have peak wavenumbers in the 1635 to 1700 cm⁻¹ wavenumber range, and the spectral intensities of the waveform components that have peak wavenumbers in the 1635 to 1655 cm⁻¹ wavenumber range. As used herein, "spectral intensity" means either peak intensity or peak area.

More specifically, calculations are performed to find the sum of the spectral intensities of the multiple waveform components that have peak wavenumbers in the 1635 to 1700 cm⁻¹ wavenumber range, and the sum of the spectral intensities of the waveform components that have peak wavenumbers in the 1635 to 1655 cm⁻¹ wavenumber range. More specifically, calculations are made to obtain the sum of the spectral intensities of a waveform component (I) that has a peak wavenumber in the 1635 to 1645 cm⁻¹ wavenumber range, a waveform component (II) that has a peak wavenumber in the 1645 to 1655 cm⁻¹ wavenumber range, a waveform component (III) that has a peak wavenumber in the 1655 to 1667.5 cm⁻¹ wavenumber range, a waveform component (IV) that has a peak wavenumber in the 1667.5 to 1677.5 cm⁻¹ wavenumber range, and a waveform component (V) that has a peak wavenumber in the 1677.5 to 1700 cm⁻¹ wavenumber range.

The calculated spectral intensities are then used to find the ratio of the spectral intensity of the waveform components that have peak wavenumbers in the 1635 to 1655 cm⁻¹ wavenumber range with respect to the spectral intensity of the waveform components that have peak wavenumbers in the 1635 to 1700 cm⁻¹ wavenumber range. More specifically, the ratio of the sum of the spectral intensities of the waveform components (I) and (II) to the sum of the spectral intensities of the waveform components (I) to (V) is calculated ((I)+(II)/(I)+(II)+(III)+(IV)+(V)).

Finally, if the calculated ratio is equal to or greater than a predetermined value, amyloid beta is determined to be contained in the substance present in the body tissue, and the result is output and presented to a user through a display. The output result may be presented as, for example, the actual numerical value of the calculated ratio, information concerning whether the numerical value is equal to or greater than or less than the predetermined value, or information concerning the presence or absence of amyloid beta.

### (2) Second Embodiment

A biological substance detection system according to Second Embodiment of the present disclosure is configured to include: a measurement device that acquires a Raman spectrum that corresponds to a substance present in a body tissue; and a spectral data analyzer that calculates the spectral intensity ratio of the C-H band and the amide I band in the Raman spectrum that corresponds to a substance in a body tissue, and that automatically determines the presence or absence of amyloid beta in the substance based on the calculated ratio. More specifically, the spectral data analyzer according to the present embodiment calculates the ratio of the spectral intensity at wavenumber 1463 cm⁻¹ with respect to the spectral intensity at wavenumber 1658 cm⁻¹ in the Raman spectrum, and determines and outputs that the substance contains amyloid beta, if the calculated ratio is less than a predetermined value.

The measurement device in the biological substance detection system according to the present embodiment is as described in First Embodiment. The CPU, memory, and hard disc of the spectral data analyzer operate with the program stored in the hard disc to perform the following steps.

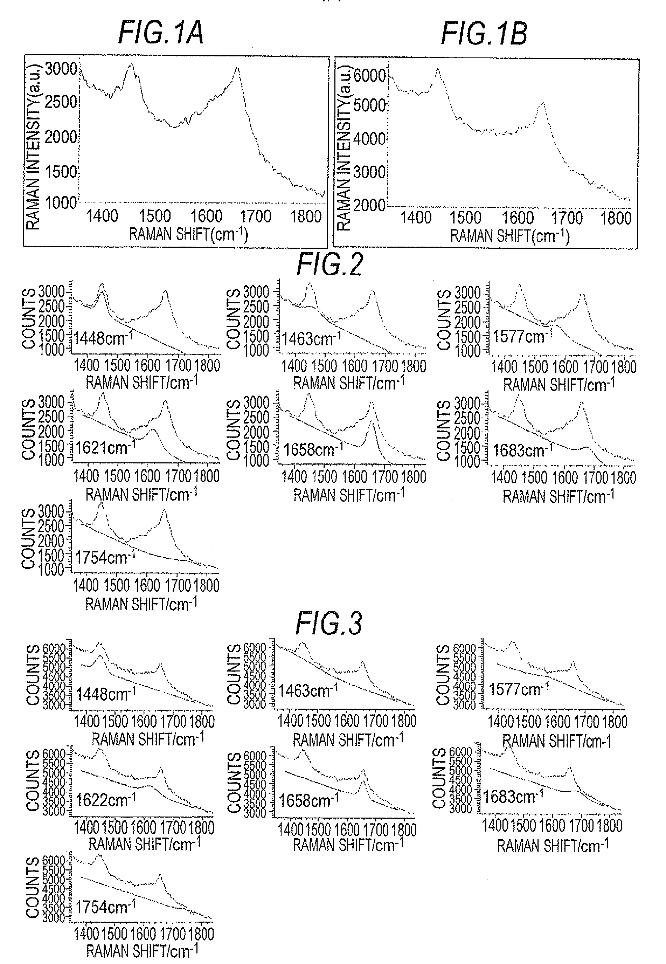

First, the C-H band spectral intensity and the amide I band spectral intensity are extracted from the output Raman spectral data from the Raman spectroscopy imaging device. FIGS. 1A and 1B represent an example of Raman spectral data. FIG. 1A represents a Raman spectrum obtained from a body tissue containing amyloid beta; FIG. 1B represents a Raman spectrum obtained from a body tissue containing no amyloid beta.

The C-H band spectral intensity and the amide I band spectral intensity may be obtained by extracting representative values, specifically, the spectral intensity at wavenumber 1658 cm⁻¹, and the spectral intensity at wavenumber 1463 cm⁻¹, respectively. The Raman spectrum may be resolved into multiple waveform components by curve fitting to extract the spectral intensities of the waveform components that have peak wavenumbers at wavenumbers 1658 cm⁻¹ and 1463 cm⁻¹. FIG. 2 and FIG. 3 represent an example of waveform components separated by curve fitting. FIG. 2 represents an example of waveform components of a Raman spectrum obtained from a body tissue containing amyloid beta. FIG. 3 represents an example of waveform components of a Raman spectrum obtained from a body tissue containing no amyloid beta.

The calculated spectral intensities are then used to calculate the ratio of the spectral intensity at wavenumber 1463 cm⁻¹ with respect to the spectral intensity at wavenumber 1658 cm⁻¹ (1463 cm⁻¹/1658 cm⁻¹).

If the calculated ratio is less than the predetermined value, amyloid beta is determined to be contained in the substance present in the body tissue, and the result is output and presented to a user through, for example, a display. The output result may be presented as, for example, the actual numerical value of the calculated ratio, information concerning whether the numerical value is less than or equal to or greater than the predetermined value, or information concerning the presence or absence of amyloid beta.

With the biological substance detection system according to the present disclosure, the amyloid beta present in a body tissue can be noninvasively and accurately detected based on the calculated ratio.

### 2. Biological Substance Detection Method

A biological substance detection method according to the present disclosure includes a procedure that corresponds to the steps performed by the biological substance detection system. Specifically, the biological substance detection method includes calculating the spectral intensity ratio of the C-H band and the amide I band of a Raman spectrum that corresponds to a substance present in a body tissue, or resolving the amide I band of the Raman spectrum into multiple waveform components by curve fitting, and calculating the ratio of (i) the spectral intensities of the waveform components that have peak wavenumbers in the 1635 to 1700 cm⁻¹ wavenumber range and (ii) the spectral intensities of the waveform components that have peak wavenumbers in the 1635 to 1655 cm⁻¹ wavenumber range; and determining the presence or absence of amyloid beta in the substance based on the calculated ratio.

The biological substance detection method may further include acquiring as background data the Raman spectrum that corresponds to a substance present in an untargeted region of the body tissue. By subtracting the spectral data (background data) obtained from the untargeted region from the spectral data obtained from the targeted region of the body tissue, the accuracy of the ratio calculation can be increased, and the presence or absence of amyloid beta can be determined more accurately.

The spectral intensity of the waveform component can be calculated as the peak intensity at the peak wavenumber or as the peak area of the peak wavenumber band in each waveform component. The peak intensity at the peak wavenumber or the peak area of the peak wavenumber band can be treated as the same when the Raman spectroscopy imaging device has a high resolution.

### Examples

### <Test Example 1: Detection 1 of Retina Macular Region Amyloid Beta>

### 1. Acquisition of Raman Spectra

Raman spectra corresponding to a substance present in the back of eye were acquired according to the following method.
(1) Apparatus: Laser Raman spectromicroscope (inVia; Renishaw)
(2) Measurement conditions: laser wavelength 532 nm; laser intensity 50 mW, spectrum-acquiring wavenumber range 1500 to 1800 cm⁻¹
(3) Measurement sample: An ultrafine glass pipette was inserted into the eye ball of ICR mice (8 weeks of age, male) or C57B6J mice (8 weeks of age, male) under a microscope, and an amyloid beta solution was injected immediately beneath the retina. The amyloid beta solution was prepared by dissolving Human 1-40 Amyloide-Beta Peptide (Peptide Institute, Inc.) or Human 1-42 Amyloide-Beta Peptide (GL Lab) in a phosphate buffer (pH 7.4) at a final concentration of 100 µM, and used after shaking at 37°C for at least 3 days. The amyloid beta peptides were dispersed as aggregates in the amyloid beta solution. The mice were euthanized after 2 hours to 1 day, and the eye ball was removed and used as a measurement sample.

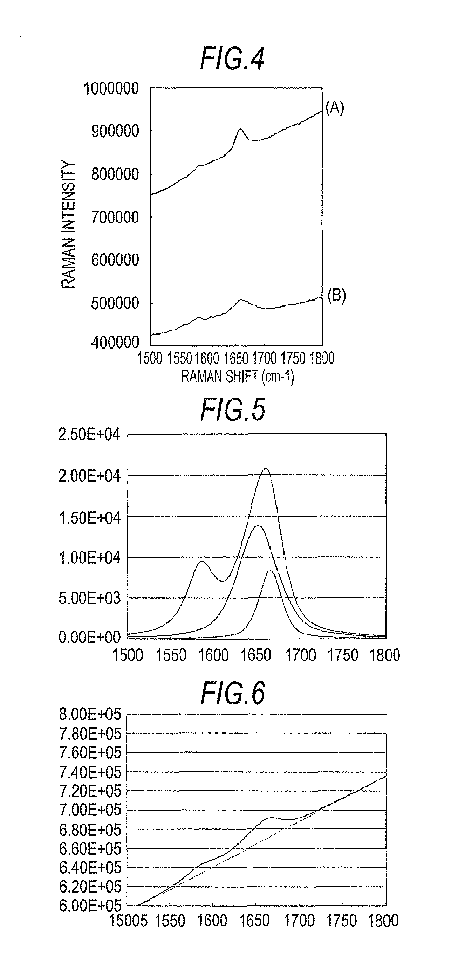

FIG. 4 represents the resulting Raman spectra. In the figure, the vertical and horizontal axes represent spectral intensity and Raman shift, respectively. The symbol (A) represents a spectrum obtained from the tested eye to which the amyloid beta solution was injected, and the symbol (B) represents a spectrum obtained from a control eye to which the amyloid beta solution was not injected.

### 2. Curve Fitting

The amide I vibrational band (1600-1700 cm⁻¹) of the Raman spectrum was resolved into multiple waveform components by curve fitting. Curve fitting was performed using commercially available software.

FIG. 5 represents the waveform components obtained by resolving the amide I vibrational band of the Raman spectrum of the tested eye.

FIG. 6 represents a waveform component obtained by resolving the amide I vibrational band of the Raman spectrum of the control eye. Unlike the control eye, the resolved waveform components are multiple in the tested eye.

### 3. Determining the Presence or Absence of Amyloid Beta

The resolved waveform components were divided into five waveform components (I) to (V) according to the wavenumber range of the peak wavenumber, and the peak wavenumber spectral intensity in each waveform component was extracted.
Wavenumber range (I): 1635 to 1645 cm⁻¹: Waveform component (I)
Wavenumber range (II) : 1645 to 1655 cm⁻¹: Waveform component (II)
Wavenumber range (III): 1655 to 1667.5 cm⁻¹: Waveform component (III)
Wavenumber range (IV): 1667.5 to 1677.5 cm⁻¹: Waveform component (IV)
Wavenumber range (V) : 1677.5 to 1700 cm⁻¹: Waveform component (V)

Then, the ratio of the sum of the spectral intensities of the waveform components (I) and (II) with respect to the sum of the spectral intensities of the waveform components (I) to (V) was calculated for the tested eye and the control eye. The mean values of ten samples for the tested eye and the control eye are presented in Table 1 below.

**Table 1**

| | Ratio (I)+(II)/(I)+(II)+(III)+(IV)+(V) |
|---|---|
| Tested Eye | 0.312 |
| Control eye | 0 |

As can be seen in Table 1, the ratio of the sum of the spectral intensities of the waveform components (I) and (II) is higher in the tested eye than in the control eye. The fraction of the sum of the spectral intensities of the waveform components (I) and (II) in all spectral intensities was 0.3 or greater in the tested eye, a value significantly higher than the ratio 0 in the control eye.

These results demonstrate that the amyloid beta can be determined as being contained in the retina when the fraction of the spectral intensities of the waveform components (I) and (II) in all spectral intensities is equal to or greater than a predetermined value (here, for example, 0.1 or more, preferably 0.2 or more, more preferably 0.3 or more).

### <Test Example 2: Detection 2 of Retina Macular Region Amyloid Beta>

### 1. Acquisition of Raman spectra

Raman spectra corresponding to a substance present in the back of eye were acquired according to the following method.
(1) Apparatus: Laser Roman spectromicroscope (inVia, Renishaw)
(2) Measurement conditions: Laser wavelength 785 nm; laser intensity 1 mW; spectrum-acquiring wavenumber range 1000 to 2000 cm⁻¹
(3) Measurement sample: An ultrafine glass pipette was inserted into the eye ball of ICR mice (8 weeks of age, male) under a microscope, and an amyloid beta solution was injected immediately beneath the retina. The amyloid beta solution was prepared by dissolving Human 1-40 Amyloide-Beta Peptide (Peptide Institute, Inc.) or Human 1-42 Amyloide-Beta Peptide (GL Lab) in a phosphate buffer (pH 7.4) at a final concentration of 100 µM. The amyloid beta peptides were dispersed in the amyloid beta solution without being aggregated. The mice were euthanized after 2 hours to 1 day, and the eye ball was removed and used as a measurement sample.

### 2. Curve Fitting

The Raman spectrum was resolved into multiple waveform components by curve fitting, and calculations were performed to find the spectral intensities of the C-H band (wavenumber 1658 cm⁻¹) and the amide I band (wavenumber 1463 cm⁻¹), and the ratio of these spectral intensities (1463 cm⁻¹/1658 cm⁻¹) was calculated.

### 3. Determining the Presence or Absence of Amyloid Beta

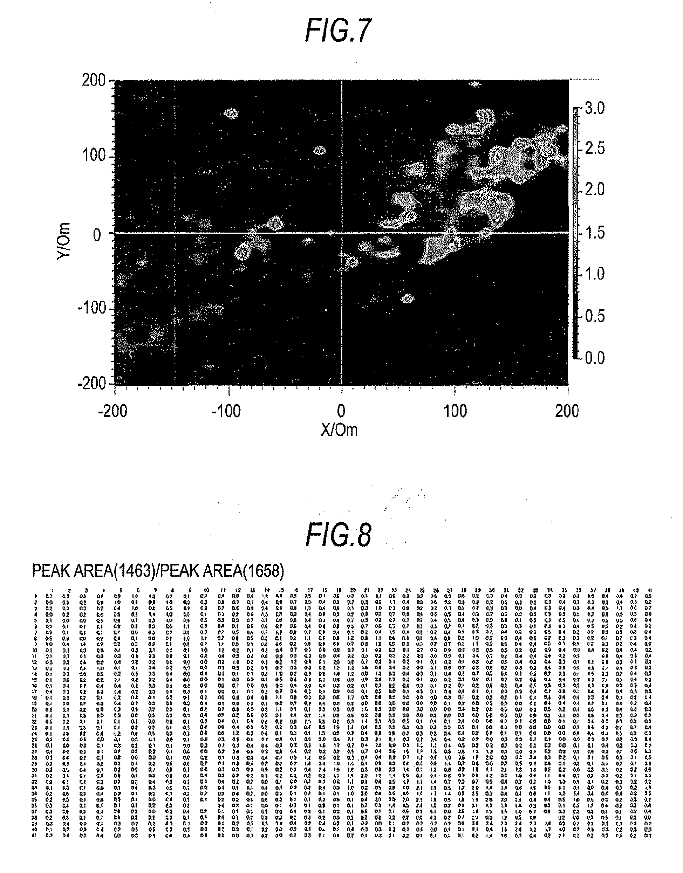

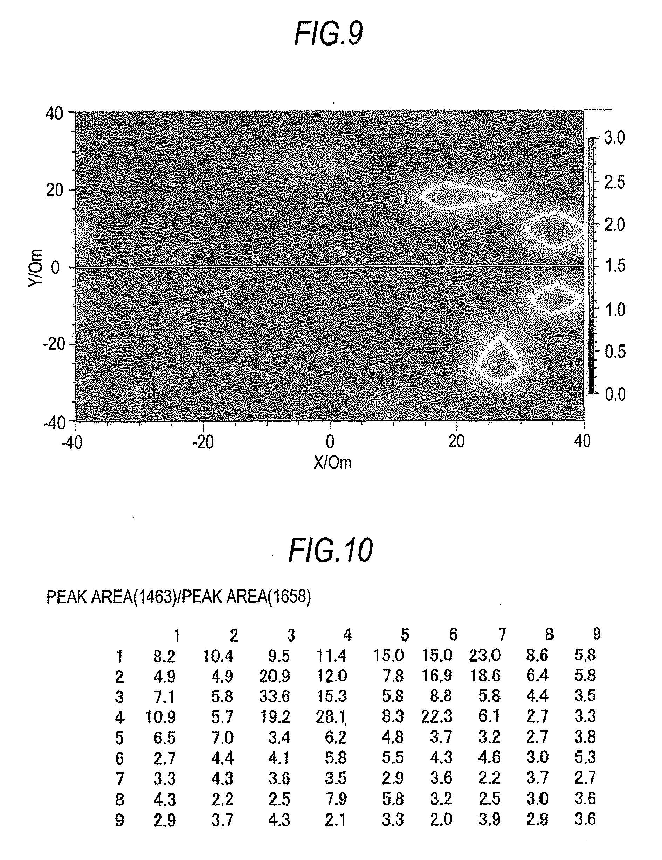

FIG. 7 to FIG. 10 show images produced from the calculated ratio, and matrices representing the mean values of the 1463 cm⁻¹/1658 cm⁻¹ ratio in each region of the images. FIGS. 7 and 8 represent the Raman spectroscopic image and the matrix, respectively, of the tested eye to which the amyloid beta solution was injected. FIGS. 9 and 10 represent the Raman spectroscopic image and the matrix, respectively, of the control eye to which the amyloid beta solution was not injected.

As shown in the matrices of FIGS. 8 and 10, the regions representing the 1463 cm⁻¹/1658 cm⁻¹ ratio of less than 1.0 occupy the majority of the matrix in the tested eye, whereas the ratio was 1.0 or more in all regions in the control eye.

The result demonstrates that the amyloid beta can be determined as being present in the retina when the spectral intensity ratio of the C-H band and the amide I band is less than a predetermined value. (For example, when the 1463 cm⁻¹/1658 cm⁻¹ ratio is less than 1.0, preferably less than 0.5, more preferably less than 0.1.) Note that the same result was obtained with the ratio obtained by directly extracting the spectral intensities at wavenumbers 1658 cm⁻¹ and 1463 cm⁻¹ from the Raman spectrum without curve fitting.

### <Test Example 3: Detection of Cerebral Amyloid Beta>

Raman spectra corresponding to a substance present in a cerebral tissue were acquired according to the following method.
(1) Apparatus: Laser Raman spectromicroscope (inVia; Renishaw), water immersion lens 60x (NA 1.0; OLYMPUS LUMPlanFIN 60xW)
(2) Measurement conditions: Laser wavelength 785 nm, laser intensity 3 mW, spectrum-acquiring wavenumber range 400 to 1900 cm⁻¹
(3) Measurement sample: Cerebrum was removed from transgenic mice expressing APP in excess (Correlative memory deficits, Abeta elevation, and amyloid plaques in transgenic mice, Science.1996, 4, 274(5284), 99-102), and frozen in liquid nitrogen. Frozen sections were prepared in 10-µm thicknesses along the median line, and placed on a gold vapor-deposited substrate.

The frozen sections were immunofluorescence-stained using antibodies (6E10) specific to amyloid beta. As a result, large numbers of amyloid beta plaques were confirmed in the cerebral cortex and the hippocampus. Amyloid beta plaques were also confirmed by dyeing that used a dye (thioflavin T) specific to amyloid beta.

The sample after the thioflavin T dyeing was placed on a gold vapor-deposited substrate, and the Raman scattering spectrum was measured. Measurement was made at each point separated by 3 µm. A scattering peak was arbitrarily selected, and a Raman scattering image was produced using the spatial distribution of the Raman shift or intensity. As a result, a band that originates in the secondary amine of the thioflavin T, and the amide I band were specified in the same region of the image, making it possible to visualize the amyloid beta plaques on the Raman scattering image.

The spectral data analyzer and the biological substance detection system of the embodiments of the present disclosure can be used for the noninvasive detection of a substance in a body tissue. The spectral data analyzer according to the embodiment of the present disclosure, among others, can thus be used for the early diagnosis or the treatment of disease conditions associated with a specific substance.

The present disclosure contains subject matter related to those disclosed in Japanese Priority Patent Applications JP 2010-151721 and JP 2011-037790 filed in the Japan Patent Office on July 2, 2010 and February 24, 2011, respectively, the entire contents of which is hereby incorporated by reference.

In so far as the embodiments of the invention described above are implemented, at least in part, using software-controlled data processing apparatus, it will be appreciated that a computer program providing such software control and a transmission, storage or other medium by which such a computer program is provided are envisaged as aspects of the present invention.

Although particular embodiments have been described herein, it will be appreciated that the invention is not limited thereto and that many modifications and additions thereto may be made within the scope of the invention. For example, various combinations of the features of the following dependent claims can be made with the features of the independent claims without departing from the scope of the present invention.

## Claims

1. A spectral data analyzer that:
calculates the spectral intensity ratio of a C-H band and an amide I band in a Raman spectrum that corresponds to a substance present in a body tissue; and
automatically determines the presence or absence of amyloid beta in the substance based on the calculated ratio.

2. The spectral data analyzer according to claim 1, wherein the spectral intensity ratio is the ratio of spectral intensities at wavenumbers 1463 cm⁻¹ and 1658 cm⁻¹ in the Raman spectrum.

3. The spectral data analyzer according to claim 2, wherein the ratio is calculated from multiple waveform components resolved from the Raman spectrum by curve fitting.

4. A spectral data analyzer that:
resolves an amide I band of a Raman spectrum corresponding to a substance present in a body tissue into multiple waveform components by curve fitting, and calculates the ratio of (i) spectral intensities of waveform components that have peak wavenumbers in a 1635 to 1700 cm⁻¹ wavenumber range and (ii) spectral intensities of waveform components that have peak wavenumbers in a 1635 to 1655 cm⁻¹ wavenumber range; and
automatically determines the presence or absence of amyloid beta in the substance based on the calculated ratio.

5. A biological substance detection system comprising:
a measurement device that acquires a Raman spectrum that corresponds to a substance present in a body tissue; and
the spectral data analyzer of claim 4.

6. A biological substance detection system comprising:
a measurement device that acquires a Raman spectrum that corresponds to a substance present in a body tissue; and
the spectral data analyzer of claim 3.

7. A biological substance detection method comprising:
calculating the spectral intensity ratio of a C-H band and an amide I band in a Raman spectrum that corresponds to a substance present in a body tissue; and
determining the presence or absence of amyloid beta in the substance based on the calculated ratio.

8. A biological substance detection method comprising:
resolving an amide I band of a Raman spectrum corresponding to a substance present in a body tissue into multiple waveform components by curve fitting, and calculating the ratio of (i) spectral intensities of waveform components that have peak wavenumbers in a 1635 to 1700 cm⁻¹ wavenumber range and (ii) spectral intensities of waveform components that have peak wavenumbers in a 1635 to 1655 cm⁻¹ wavenumber range; and
determining the presence or absence of amyloid beta in the substance based on the calculated ratio.
